# EUROPEAN PATENT SPECIFICATION

(11) **EP 0 408 389 B1**
(45) Date of publication and mention of the grant of the patent: **14.09.1994**
(21) Application number: 90307707.1
(22) Date of filing: 13.07.1990
(51) Int. Cl.: A61M 25/02

(54) **Device usable as a catheter holder**
Als Katheterhalterung verwendbare Vorrichtung
Dispositif utilisable comme support d'un cathéter

(30) Priority: 14.07.1989 GB 8916191; 09.08.1989 GB 8918171
(43) Date of publication of application: 16.01.1991
(60) Divisional application: 93113064.5
(73) Proprietor: E.R. SQUIBB & SONS, INC., Princeton New Jersey 08543-4000 (US)
(72) Inventor: Plass, Ronald Andrew, Lingfield, Sussex (GB); Hollands, Keith G.M., Sompting,, Sussex (GB)
(74) Representative: Cook, Anthony John

(56) References cited:
- EP-A- 0 122 344
- GB-A- 2 211 417
- US-A- 3 924 636
- US-A- 4 074 397
- US-A- 4 333 468

## Description

This invention relates to a catheter holder.

As a result of various medical procedures, catheters often are employed to drain body fluids or administer substances. Various devices have been developed for the purpose of securing a catheter or other tubing to the body of the patient to prevent accidental dislodgement. Devices which rely on an adhesive attachment to the catheter or tubing are shown, for example, by Mellor in U.S. Patent 3,826,254, Haerr in U.S. Patent 4,122,857, Brown in U.S. Patent 4,484,914, Moseley in U.S. Patent 4,460,356, Vaillancourt in U.S. Patents 4,801,296 and 4,830,914, and Muller in European Patent Application 206,558. Hesketh in U.K. Patent Application 2,211,417 shows a catheter holder in which a rectangular tab is secured to a pad, the tab and the pad each being a combination of a medical grade adhesive layer and a non-adhesive embossed polyethylene sheet. The polyethylene sheets are heat-welded together over a small area in such a way as to permit folding movement of the tab relative to the pad. The upper surface of the tab is adhesive and is covered, prior to use of the article, with release paper.

U.S. Patent No. 4,333,468 to Geist, which is forming the closest prior art, discloses a mesentery tube holder apparatus. As seen in Geist's Figure 4, an elongated moderately pliable base carries a pair of generally rectangular flaps 31, 32 upon the inner surfaces of which are respective adhesive layers 35. The free ends 31d, 32d, of Geist's flaps are free of adhesive.

According to the invention, there is provided a catheter holder which comprises a pad of medical grade adhesive material having one surface for attachment to the skin of the wearer, tape means secured to the other surface of the pad and providing a pair of tape sections which extend separately from said other surface and which are aligned with each other for receiving a catheter therebetween, each said tape section having an inner and an outer surface, and multiple use adhesive on the inner surface of a first tape section which faces towards the second tape section such that the facing inner surfaces of the tape sections can be temporarily adhered together outwardly of the catheter to thereby hold it closely adjacent said other surface of the pad, the distal ends of the tape sections being free of adhesive so that the distal ends of the tape sections can be used to facilitate peeling the tape sections apart, characterised in that a substantial part of the inner surface of the second tape section is free of adhesive and in that said adhesive also extends along said second tape section to a sufficient extent to adhesively engage the catheter when the catheter is secured by the tape sections.

Optionally, the pad may have an opening or hole near the point of attachment of the tapes, so that the catheter can be threaded through the pad. Alternatively, the pad may be slit inwardly from its edge with the slit joining a hole. This arrangement enables a catheter to be engaged with the device without a threading operation. It is often necessary to avoid threading, e.g. where one end of the catheter is attached internally of the wearer and the other end is attached to a container for the discharge of exudate.

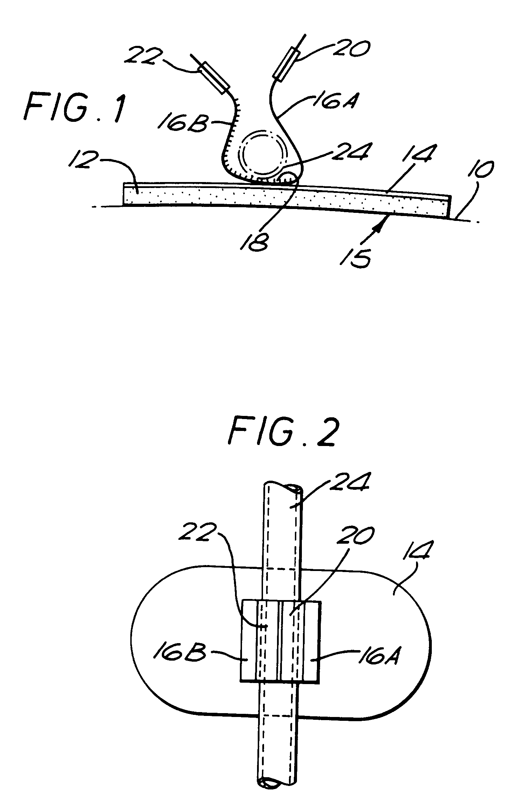

An important advantage of the disclosed embodiment of this invention is that the catheter holder can accommodate many different sizes of catheter. This is in marked contrast to most if not all of the catheter holders currently available. These suffer from the disadvantage that, when a large catheter is to be used, there is the need to search for and obtain a catheter holder of a size precisely appropriate to the catheter concerned.
Figure 1 is a side elevation of a catheter holder in accordance with the invention showing tapes thereof separated;
Figure 2 is a plan view of the catheter holder shown in Figure 1 but showing the tapes stuck together;
The device illustrated in Figures 1 and 2 includes a pad of medical grade adhesive material 12. The pad 12 has on its top or free surface a film 14 of a synthetic plastics material. The pad 12 is shown as applied to the skin of a wearer, the skin being denoted by 10. Prior to application, the surface 15 of the pad 12 is covered by a strippable release paper or sheet.

Any of the known medical grade pressure sensitive adhesives can be employed. Preferably, adhesive material 12 is formulated by blending one or more water soluble or swellable hydrocolloids with a polyisobutylene or a mixture of polyisobutylenes or a mixture of one or more polyisobutylenes and one or more non-acrylic elastomers. Other materials can be included within the adhesive formulations such as mineral oil, tackifiers, antioxidants, cohesive strengthening agents, and pharmaceutically active materials such as anti-inflammatory agents, antiseptics, or materials having skin healing or soothing properties. Suitable adhesive formulations are taught by Chen in U.S. Patent 3,339,546, Chen et al. in U.S. Patent 4,192,785, Pawelchak et al. in U.S. Patent 4,393,080, Doyle et al. in U.S. Patent 4,551,490, and by Keyes et al. in U.S. Patent 4,762,738. As disclosed in these references, suitable water soluble and water swellable hydrocolloids include sodium carboxymethylcellulose, pectin, gelatin, guar gum, locust bean gum, gum karaya, and mixtures thereof. Suitable cohesive strengthening agents include water-insoluble cross-linked sodium carboxymethylcellulose, water-insoluble cross-linked dextran, etc. Suitable non-acrylic elastomers include butyl rubber and styrene radial or block copolymers such as styrene-butadiene-styrene (S-B-S) and styrene-isoprene-styrene (S-I-S) block type copolymers.

A pair of tape or strip members 16A, 16B are attached to the film 14 in any suitable manner. These tapes may be synthetic plastics strips. They may be formed by a single strip of material fastened to the film 14 at an intermediate region. A non-adhesive grippable tab 20 is carried by the free end of the tape 16A and a similar tab 22 is carried by the tape member 16B. Although referred to as two separate tapes, it will be understood that equally efficient results can be obtained by using a single tape member having two free ends. A multi-use adhesive 18 is applied to a length of the inner surface of the tape member 16B, covering all or most of the region where the tape is attached to the film 14 and extending up nearly to the gripping tab 22. The corresponding inner surface of the tap 16A is free of adhesive. With this arrangement, the tape 16A and 16B can be pressed together and remain stuck together but can readily be separated by pulling apart the tabs 20 and 22. The sticking together and separating operations can be successfully repeated in accordance with the characteristics of the chosen multi-use adhesive.

Multi-use refers to the ability of the adhesive to retain its tack after it is peeled from a surface so that it can be refastened. Such adhesives are commercially available, for example from the 3M Company, and are employed in various products such as adhesive tabs on diapers.

In use, a length of a catheter 24 is placed between the tape members 16A and 16B and these are then pinched together to tightly surround and enclose the catheter 24. The adhesive on the tape 16B attaches to the catheter and prevents the catheter 24 being moved in the direction of its length relative to the catheter holder, but the catheter can readily be removed by separating the tape members 16A and 16B. In addition, if desired the medical grade adhesive pad 12 can be removed from the skin 10 without releasing the catheter 24 from the catheter holder.

Although not shown in Figures 1 and 2, the pad 12 may have a slit therein. This is useful in the case that the catheter 24 extends into the body through a surgically made aperture, because the medical grade adhesive pad can then be slid into position by moving it relative to the catheter so that the catheter slides into the end of the slot in the pad 12. Thereafter the pad 12 is stuck to the skin 10 and the tape members are closed to securely retain the catheter as described above.

Although not shown in Figures 1 and 2, the adhesive on the tapes 16A, 16B is normally covered with a strippable protective release paper or film, to avoid the adhesive picking up dirt or being degraded prior to use.

It will be understood that modifications may be made to the catheter holders particularly described and illustrated herein, without departing from the invention. For example, the tape members need not be of synthetics plastics material but could be of other material which could carry a suitable multi-use adhesive. The shape and positioning of the tape members on the pad 12 need not be precisely as illustrated. In certain circumstances it may be practical to dispense with the cover film 14. Gripping tabs other than those of the kind illustrated in the Figures may be utilized. Also, the holders are useful for securing other types of tubing, wires, etc., in addition to drainage catheters, to the patient as may be required.

The present invention may find application otherwise than as a catheter holder. In particular, a device substantially as disclosed herein but employing an adhesive pad 12 (which need not necessarily be hydrocolloid-based) can be used to removably attach the edges of medical drapes to the skin or clothing of a patient undergoing surgery, or can be used to anchor medical drapes in a desired position relative to a patient. The claims herein are to be interpreted in a manner consistent with this.

## Claims

1. A catheter holder which comprises a pad (12) of medical grade adhesive material having one surface (15) for attachment to the skin of the wearer, tape means (16) secured to the other surface of the pad and providing a pair of tape sections (16A,16B) which extend separately from said other surface and which are aligned with each other for receiving a catheter (24) therebetween, each said tape section having an inner and an outer surface, and multiple use adhesive (18) on the inner surface of a first tape section (16B) which faces towards the second tape section (16A) such that the facing inner surfaces of the tape sections can be temporarily adhered together outwardly of the catheter to thereby hold it closely adjacent said other surface of the pad, the distal ends of the tape sections (20,22) being free of adhesive so that the distal ends of the tape sections (20,22) can be used to facilitate peeling the tape sections apart, characterised in that a substantial part of the inner surface of the second tape section (16A) is free of adhesive and in that said adhesive also extends along said second tape section (16A) to a sufficient extent to adhesively engage the catheter when the catheter is secured by the tape sections (16A,16B).

2. A catheter holder according to claim 1 in which the pad has a through hole near to the point of attachment of the tape section, so that the catheter can be threaded through the pad.

3. A catheter holder according to claim 2 in which the pad is slit inwardly from its edge with the slit joining said hole.

4. A catheter holder according to any preceeding claim in which the medical grade adhesive material is a blend comprising one or more water soluble or swellable hydrocolloids with a polyisobutylene or a mixture of polyisobutylenes or a mixture of one or more polyisobutylenes and one ore more non-acrylic elastomers.

## Patentansprüche

1. Katheterhalterung mit einem Polster (12) aus medizinischem Haftmaterial mit einer Oberfläche (15) zur Befestigung auf der Haut des Trägers, einer Bandeinrichtung (16), die an der anderen Oberfläche des Polsters befestigt ist und ein Paar Bandabschnitte (16A, 16B) hat, die einzeln von der anderen Oberfläche verlaufen und die zueinander ausgerichtet sind, um zwischen ihnen einen Katheter (24) aufzunehmen, wobei jeder Bandabschnitt eine Innen- und eine Außenfläche sowie mehrfach verwendbares Haftmittel (18) auf der Innenfläche eines ersten Bandabschnitts (16B) hat, der dem zweiten Bandabschnitt (16A) so gegenüberliegt, daß die gegenüberliegenden Innenflächen der Bandabschnitte zeitweilig außerhalb des Katheters haftend zusammengefügt werden können, um diesen dadurch eng an der anderen Oberfläche des Polsters festzuhalten, und die entfernten Enden (20, 22) der Bandabschnitte haftmittelfrei sind, so daß die entfernten Enden (20, 22) der Bandabschnitte dazu verwendet werden können, das Auseinanderziehen der Bandabschnitte zu erleichtern, dadurch gekennzeichnet, daß ein wesentlicher Teil der Innenfläche des zweiten Bandabschnitts (16A) haftmittelfrei ist und daß das Haftmittel auch in ausreichendem Maß entlang dem zweiten Bandabschnitt (16A) verläuft, um den Katheter haftend einzubinden, wenn der Katheter durch die Bandabschnitte (16A, 16B) befestigt ist.

2. Katheterhalterung nach Anspruch 1, wobei das Polster ein Durchgangsloch in der Nähe des Befestigungspunktes des Bandabschnitts aufweist, so daß der Katheter durch das Polster gezogen werden kann.

3. Katheterhalterung nach Anspruch 2, wobei das Polster im Inneren mit einem Schlitz von seiner Kante aus versehen ist und der Schlitz in das Loch mündet.

4. Katheterhalterung nach einem der vorausgegangenen Ansprüche, wobei das medizinische Haftmaterial ein Gemisch aus einem oder mehreren wasserlöslichen oder -quellbaren Hydrokolloiden mit Polyisobutylen oder einer Mischung aus Polyisobutylenen oder einer Mischung aus einem oder mehreren Polyisobutylenen und einem oder mehreren Nichtakrylelastomeren ist.

## Revendications

1. Porte-cathéter comprenant un tampon (12) de matière adhésive de qualité médicale dont une surface (15) est destinée à être fixée à la peau du porteur, un moyen (16) en forme de bande ou de ruban fixé à l'autre surface du tampon et présentant deux parties (16A,16B) qui partent séparément de ladite autre surface et qui se trouvent en regard l'une de l'autre pour recevoir un cathéter (24) entre elles, chacune desdites parties de bande ayant une surface intérieure et une surface extérieure, et un adhésif multi-usage (18) sur la surface intérieure d'une première partie de bande (16B), qui est tourné vers la seconde partie de bande (16A) de telle sorte que les surfaces intérieures en regard des parties de bande peuvent être temporairement collées l'une à l'autre extérieurement au cathéter de façon à le maintenir tout contre ladite autre surface du tampon, les extrémités distales (20,22) des parties de bande étant dépourvues d'adhésif pour que les extrémités distales (20,22) des parties de bande puissent servir à faciliter le décollement des parties de bande, caractérisé en ce qu'une partie notable de la surface intérieure de la seconde partie de bande (16A) est dépourvue d'adhésif, et en ce que ledit adhésif s'étend aussi le long de ladite seconde partie de bande (16A) dans une mesure suffisante pour venir en contact adhésif avec le cathéter quand le cathéter est immobilisé par les parties de bande (16A,16B).

2. Porte-cathéter selon la revendication 1, dans lequel le tampon est traversé par un trou à proximité du point de fixation de la partie de bande, pour que le cathéter puisse être enfilé dans le tampon.

3. Porte-cathéter selon la revendication 2, dans lequel le tampon est fendu vers l'intérieur à partir de son bord, la fente rejoignant ledit trou.

4. Porte-cathéter selon l'une quelconque des revendications précédentes, dans lequel la matière adhésive de qualité médicale est un mélange comprenant un ou plusieurs hydrocolloîdes solubles dans l'eau ou gonflant dans l'eau, avec du polyisobutylène, ou un mél ange de polyisobutylènes, ou un mélange de un ou plusieurs polyisobutylènes et de un ou plusieurs élastomères non acryliques.
